(19) Europäisches Patentamt
European Patent Office
Office européen des brevets

(11) Publication number: 0 183 346
A2

(12) # EUROPEAN PATENT APPLICATION

(21) Application number: 85306582.9

(22) Date of filing: 17.09.85

(51) Int. Cl.[4]: C01B 33/20 , C07C 2/12

(30) Priority: 18.09.84 US 651748

(43) Date of publication of application:
04.06.86 Bulletin 86/23

(84) Designated Contracting States:
BE DE FR GB IT NL SE

(71) Applicant: MOBIL OIL CORPORATION
150 East 42nd Street
New York New York 10017(US)

(72) Inventor: Chang, Clarence Dayton
11 Murray Place
Princeton New Jersey 08540(US)
Inventor: Chu, Cynthia Ting-Wah
283 Westcott Boulevard
Pennington New Jersey 08534(US)
Inventor: Kuehl, Guenter Hinrich
1956 Cardinal Lake Drive
Cherry Hill New Jersey 08003(US)

(74) Representative: West, Alan Harry
Mobil Court 3 Clements Inn
London WC2A 2EB(GB)

(54) **Iron-containing silicate having ZSM-5 crystalline structure, its method of preparation, and its use in hydrocarbon conversion.**

(57) A crystalline iron-containing silicate has the X-ray diffraction pattern of ZSM-5 and a crystallographic lattice containing silicon and iron, wherein at least 60% by weight of the iron contained by said silicate is in positions of tetrahedral substitution in the lattice and wherein the molar ratio of $SiO_2$ to $Fe_2O_3$ in said crystalline iron containing silicate is at least 30.

EP 0 183 346 A2

# IRON-CONTAINING SILICATE HAVING ZSM-5 CRYSTALLINE STRUCTURE, ITS METHOD OF PREPARATION, AND ITS USE IN HYDROCARBON CONVERSIONS

The invention is directed to a crystalline ferrosilicate, having an X-ray diffraction pattern characteristic of ZSM-5 and to its production. These ferrosilicate compositions are useful as catalyst components for hydrocarbon conversions, for example, for oligomerization-polymerization of $C_1$-C olefins.

Naturally occurring and synthetic zeolites have been demonstrated to exhibit catalytic properties for various types of hydrocarbon conversions. Certain zeolites are ordered porous crystalline aluminosilicates having definite crystalline structure as determined by X-ray diffraction studies. Such zeolites have pores of uniform size which are uniquely determined by the unit structure of the crystal. The zeolites are referred to as "molecular sieves" because the uniform pore size of a zeolite material may allow it to selectively adsorb molecules of certain dimensions and shapes.

By way of background, one authority has described the zeolites structurally, as "framework" aluminosilicates which are based on an infinitely extending three-dimensional network of $AlO_4$ and $SiO_4$ tetrahedra linked to each other by sharing all of the oxygen atoms. Furthermore, the same authority indicates that zeolites may be represented by the empirical formula

$$M_{2/n}O.Al_2O_3.xSiO_2.yH_2O$$

In the empirical formula, x is equal to or greater than 2, since $AlO_4$ tetrahedra are joined only to $SiO_4$ tetrahedra, the ratio of the total of silicon and aluminum atoms to oxygen atoms is 1:2 and n is the valence of a cation designated M. (Breck, ZEOLITE MOLECULAR SIEVES, John Wiley & Sons, New York p. 5 (1974)).

The prior art describes a variety of synthetic zeolites, which have come to be designated by letter or other convenient symbols. The silicon/aluminum atomic ratio of a given zeolite is often variable. Moreover,in some zeolites, the upper limit of the silicon/aluminum atomic ratio is unbounded. ZSM-5 is one such example wherein the silicon/aluminum atomic ratio is at least 2.5 and up to infinity. Crystalline silicate ZSM-5 and its conventional preparation are taught by U.S. Patent 3,702,886, whereas United States Patent No. 3,941,871, reissued as RE. 29,948, discloses a porous crystalline silicate made from a reaction mixture containing no deliberately added aluminum and exhibiting the X-ray diffraction pattern characteristic of ZSM-5.

Various patents describe inclusion of elements other than silicon and aluminum in the preparation of zeolites. For example, U.S. Patent Nos. 3,530,064, 4,208,305 and 4,238,318 describe the preparation of crystalline silicates in the presence of iron to thereby produce silicates containing iron. However, a major proportion of the iron is external to the crystalline framework.

In one aspect, the invention is directed to a crystalline iron containing silicate having the X-ray diffraction pattern of ZSM-5 and a crystallographic lattice containing silicon and iron, wherein at least 60% by weight of the iron contained by the silicate occupies positions of tetrahedral substitution in the lattice and wherein the molar ratio of $SiO_2$ to $Fe_2O_3$ in said crystalline iron containing silicate is at least 30.

Preferably, at least 80% by weight of the iron contained by the silicate occupies positions of tetrahedral substitution in the lattice.

In a further aspect, the invention is directed to a method for preparing the crystalline iron-containing silicate of said one aspect comprising the steps of:

(a) providing a crystallization reaction mixture which includes a source of $Fe_2O_3$, a source of $SiO_2$ and water, wherein said source of $Fe_2O_3$ comprises a complex of $Fe^{3+}$; and

(b) maintaining said crystallization reaction mixture at a temperature of 80°C to 200°C at autogenous pressure and maintaining said complex of $Fe^{3+}$ in the complexed state until crystals of said ferrosilicate form.

Thus the present invention pertains to a class of silicate compositions, in which iron is a substitute for part of the silicon in the zeolite crystal lattice. In such a material, the acidity (acid strength) attributable to iron sites in the lattice is less than would be obtained if aluminum was the lattice substituent. This has the advantage, when the composition is used as a catalyst, of reducing acid catalysis of secondary reactions which are attributable to high acid strength catalysts. Moreover, products of the invention exhibit on-stream stability during catalysis.

The iron-containing siliceous material is a crystalline, three-dimensional continuous framework structure which results when the oxygen atoms in the tetrahedra are shared by tetrahedral silicon and iron atoms, and which has the following formula:

$$(1.0 \pm 0.15)\ M_{2/n}\ O.Fe_2O_3.\ \ x\ SiO_2.yH_2O$$

wherein M is cation of valence n, x is 30-500 and y is 0-40. It is, however, to be appreciated that where M is a tetrapropylammonium cation used in synthesizing the ferrosilicate, the as-crystallized material may contain an excess of the cation M above that indicated by the formula.

The preferred iron-containing siliceous material has a $SiO_2$ to $Fe_2O_3$ molar ratio at least 30, preferably 30 to 500 and most preferably 40 to 300. At least 60% by weight, and preferably at least 80% by weight, of the iron contained by the material occupies positions of tetrahedral substitution within the silica lattice thereby generating a negative charge which is balanced by the organic cations used in the synthesis. The organic cations, e.g., tetraalkylammonium, used in the synthesis may be removed by thermal decomposition and, after such calcination, may be replaced at least in part by other ions using conventional ion exchange techniques. Ions introduced to replace the original organic cations may be any that are desired so long as they can pass through the channels within the subject crystals. To the extent that iron is present, it will contribute to the ion-exchange capacity of highly siliceous crystals. Desired replacing ions are ammonium and metals of Groups I through VIII of the Periodic Table, among which the particularly preferred metal ions are those of the rare earth metals, manganese, zinc and those of Group VIII of the Periodic Table, e.g., platinum.

The iron-containing siliceous material is prepared by providing a reaction mixture containing a source of silica, an organic template or directing agent $R_4J$ wherein R is an alkyl or aryl group containing 1 to 7 carbon atoms and J an element of Group V-B, preferably a nitrogen , a source of iron ($Fe^{3+}$) oxide, and water, and typically having a composition, in terms of mole ratios of oxides, within the following ranges:

| | Broad | Preferred |
|---|---|---|
| $SiO_2/Fe_2O_3$ | 30 to 500 | 40 to 300 |
| $M_{2/n}O/[(R_4J)_2O + M_{2/n}O]$ | 0.6 to below 1.0 | 0.75 to 0.95 |
| $OH^-/SiO_2$ | 0.05 to 0.35 | 0.10 to 0.30 |
| $H_2O/[(R_4J)_2O+M_{2/n}O]$ | 25 to 150 | 35 to 100 |
| $R_4J/SiO_2$ | 0.01 to 0.30 | 0.02 to 20 |

wherein M is alkali or alkaline earth metal of valence n and maintaining the mixture at crystallization conditions until crystals of the iron-containing material are formed.

The reaction mixture can be prepared utilizing materials which supply the appropriate oxide. Such materials include oxides of silicon essentially free of alkali metal such as silicic acid, acid washed silica, tetraalkyl silicates and ammonium silicate. Complexes of trivalent iron are the preferred source of $Fe_2O_3$. In fact, it is believed that use of complexes of trivalent iron may maximize the amount of iron entering the crystal lattice by obviating precipitation of the iron as the hydroxide. Preferably, the trivalent iron is complexed by sulfate anion, but other suitable iron complexing agents are salts of gluconic acid, diethanol glycine, ethylene diamine tetraacetic acid, diethyetriaminepentaacetic acid, and citric acid.

The organic template $R_4J$ contains any element J of Group V B such as nitrogen or phosphorus, preferably nitrogen and an alkyl or aryl group R having between 1 and 7 carbon atoms with at least one R group preferably being a methyl, ethyl or propyl group. The oxide of the quaternary compound is generally supplied by introducing into the reaction mixture a composition such as the hydroxide, chloride or bromide of the tetraalkyl derivative of the desired V-B element, e.g., tetraethylammonium bromide, and tetrapropylammonium bromide. Alkylammonium cation precursors generated in situ by reaction of tertiary amines with an alkyl halide also may be used.

The quaternary compound, preferably the bromide, is provided in an amount sufficient to provide a pH for the crystallization reaction mixture within the range of 9 to 13. The final pH is preferably 10.5 to 12.5 which can be achieved by pH adjustment with, for example, concentrated ammonium hydroxide or sulfuric acid. In a typical preparation, an acid iron solution is admixed with a silicate solution by adding e.g. 25% tetrapropyl ammonium bromide solution in water to the $SiO_2$ source and heating to 100°C to dissolve the $SiO_2$ source.

As an alternative to the quaternary compound $R_4J$, hexamethylene diamine can be used as the template or directing agent.

The reaction mixture can be prepared either batchwise or continuously. It is noted that although aluminum is not deliberately added to the crystallization reaction mixture, it may be present in the final product as a result of minor amounts of aluminum impurities in the precursors of the reactants or extracted from the reaction vessel. Crystal size and crystallization time of the reactant, will vary with the nature of the reaction mixture employed and the crystallization conditions.

In all cases, synthesis of the desired crystals may be facilitated by the presence of at least 0.001 percent, preferably at least 0.10 percent and still more preferably at least 1.0 percent, seed crystals (based on total weight) of a previously prepared crystalline product.

Crystallization of the iron-containing siliceous material can be carried out at either static or stirred condition in polypropylene jars or in stainless steel autoclaves or at a temperature of 80°C to 200°C, preferably 100°C, for about 6 hours to 150 days at autogenous pressures. Thereafter, the crystals are separated from the liquid and recovered.

The resultant iron-containing ZSM-5 is almost colorless, although it contains several weight percent of iron oxide. The acid strength of the acid sites associated with this iron in the acid form of the zeolite is lower than for the corresponding alumino-silicate ZSM-5. Using the temperature at which ammonia is released from the ammonium form as a gauge for the acid strength, the peak temperature is 70°C lower than observed for the aluminum-containing form. This lower acidity can advantageously influence the reaction selectivity when the material is used as a catalyst.

The resultant iron containing silicate may then be calcined to remove the organic material and then subjected to ammonium ion exchange to produce the precursor of the catalytically active hydrogen form of the ferrosilicate. The source of the ammonium ion is not critical; thus the source can be ammonium hydroxide or an ammonium salt such as ammonium nitrate, ammonium sulfate, ammonium acetate, ammonium chloride and mixtures thereof. The pH of the solution is also not critical but generally is maintained to 7 to 9. Ammonium exchange may be conducted for a period of time ranging from 0.5 to 20 hours at a temperature ranging from ambient up to 100 °C. Calcination may then be effected to convert the ammonium exchanged ferrosilicate to its acid form by evolution of $NH_3$. Calcination may be effected at elevated temperatures, e.g. about 600°C.

It may be desirable to incorporate the iron-containing silicate of the invention in a matrix. Such matrix is useful as a binder and imparts resistance to the catalyst for temperature, pressure and velocity conditions encountered in many processes. Matrix materials include both synthetic and natural substances, for example, clays, silica and/or metal oxides. The mixture may be either naturally occurring or in the form of gelatinous precipitates, sols or gels including mixtures of silica and metal oxides. Frequently, zeolite materials have been incorporated into naturally occurring clays, e.g. bentonite and Kaolin.

In addition to the foregoing materials, the zeolite for use herein can be composited with a porous matrix material such as silica-alumina, silica-magnesia, silica-zirconia, silica-thoria, silica-beryllia, silica-titania, as well as ternary compositions such as silica-alumina-thoria, silica-alumina-zirconia, silica alumina-magnesia and silica-magnesia-zirconia. The matrix can be in the form of a cogel.

The iron-containing silicate of the invention is useful as a catalyst in organic conversion processes, in particular oligomerization of lower olefins, preferably $C_2$-$C_4$ olefins, to produce heavier hydrocarbons. Typical oligomerization conditions include a temperature of 175-375°C, preferably 230-270°C, a pressure of 1000-20000 kPa, preferably 3500-15000 kPa, and a liquid hourly space velocity of 0.1-10, preferably less than 1.

## EXAMPLES

### Example 1

Solution A:

Ferric sulfate, $Fe_2(SO_4)_3.7.1.H_2O$, 185 g, was dissolved in 2330 g of water. Concentrated sulfuric acid, 501 g, was added and the solution cooled to ambient temperature and a 30% solution of tetrapropylammonium bromide, 1755 g, was added. Finally, 325 g of anhydrous $Na_2SO_4$ was added and dissolved with stirring.

Solution B:

Q-Brand sodium silicate solution (PQ Corp.) (8.9 wt.% $Na_2O$, 28.7 wt.% $SiO_2$), 6000 g, was diluted with 3472 g of water and 17 g of Daxad 27, a dispersant, was added.

Both solutions were cooled to ambient temperature and nozzle-mixed into a 5-gal stainless steel autoclave, precharged with 172 g of water. Anhydrous sodium sulfate, 1040 g, was added, and the gelled mixture was vigorously stirred for one hour. The mixture was then heated for 6 days to 105°C for crystallization. The product was white and did not turn brown upon calcination. It gave the x-ray diffraction pattern of ZSM-5 of 95% crystallinity. The sorption capacities at 25°C were:

Cyclohexane, 20 Torr : 7.5 g/100 g

n-Hexane, 20 Torr : 11.4 g/100 g

Water, 12 Torr : 4.7 g/100 g

The chemical composition was:

84.4% $SiO_2$

0.25% $Al_2O_3$

3.14% $Fe_2O_3$

0.85% $Na_2O$

0.76% N

88.5% Ash

$SiO_2/(Al_2O_3 + Fe_2O_3) = 63.7$

$Fe/(Fe+Al) = 0.89$

Framework Fe = 88% by wt. total Fe

The ammonium-exchanged form had a (Na+N)/Al+Fe) atomic ratio of 0.90, somewhat less than the expected 1.0. Since no discoloration attributable to iron oxide was observed upon calcination, it is likely that hydronium ions were exchanged along with $NH_4+$ ions, but iron was not removed from the zeolite framework.

A reaction mixture identical to that yielding an white ferrosilicate ZSM-5 at about 100°C was crystallized for 3 days at 160°C in a pressure vessel at autogenous pressure. The product was again white and had 95% crystallinity based on an aluminosilicate ZSM-5 reference sample.

### Example 2

The following example demonstrates that a white ferrosilicate ZSM-5 can be crystallized in the virtual absence of alumina.

Tetraethylorthosilicate was hydrolyzed with a solution of 8.0 g of sodium hydroxide (97% NaOH) in 147.6 g of water. The alcohol generated was boiled off, and the solution was cooled to ambient temperature (solution A). Iron (III) sulfate, $Fe_2(SO_4)_3.7.1$ $H_2O$, was dissolved in 46.6 g of water and a solution of 10.5 g of tetrapropylammonium bromide in 24.6 g of water was added. Finally, 6.5 g of sodium sulfate, $Na_2SO_4$, was dissolved in the combined solution to yield solution B.

Solution B was then mixed with solution A. Sodium sulfate, $Na_2SO_4$, 20.8 g, was blended into the mixture until it was dissolved. The reaction mixture was then digested at 100°C in a sealed polypropylene jar until crystalline. The product was white and had an apparent crystallinity of 80%. After calcination at 550 °C, it had the following sorptive capacities, g/100 g:

Cyclohexane, 40 Torr 6.8

n-Hexane, 40 Torr 9.7

Water, 12 Torr 6.0

The chemical composition of the material dried at ambient temperature was:

78.4% $SiO_2$

20 ppm $Al_2O_3$

3.86% $Fe_2O_3$

1.35% $Na_2O$

0.79% N

85.16% Ash

$Fe/(Fe + Al) = 0.999$

$SiO_2/Al_2O_3 = 66,600$

$SiO_2/Fe_2O_3 = 54.2$

In Table I, a list of the d-spacings and relative intensities for the ferrosilicates of Examples 1 and 2 are recorded.

TABLE 1

| Example 1 | | Example 2 | |
|---|---|---|---|
| d,A | $I/I_o$ | d,A | $I/I_o$ |
| 11.26 | 22 | 11.33 | 20 |
| 10.12 | 15 | 10.16 | 15 |
| 9.07 | 2 | 9.08 | 2 |
| 7.50 | 5 | 7.52 | 4 |
| 7.14 | 2 | 7.16 | 2 |
| 6.76 | 3 | 6.78 | 2 |
| 6.41 | 6 | 6.44 | 5 |
| 6.05 | 6 | 6.06 | 6 |
| 5.75 | 5 | 5.77 | 4 |
| 5.60 | 6 | 5.60 | 6 |
| 5.41 | 1 | 5.42 | 1 |
| 5.17 | 2 | 5.19 | 2 |
| 5.04 | 3 | 5.04 | 4 |
| 4.63 | 5 | 4.65 | 5 |
| 4.49 | 2 | 4.49 | 2 |
| 4.39 | 7 | 4.39 | 8 |
| 4.29 | 8 | 4.29 | 8 |
| 4.11 | 2 | 4.11 | 3 |
| 4.02 | 6 | 4.03 | 7 |
| 3.86 | 100 | 3.86 | 100 |
| 3.77 | 29 | 3.78 | 29 |
| 3.73 | 45 | 3.74 | 45 |
| 3.66 | 29 | 3.67 | 27 |
| 3.50 | 4 | | |
| 3.46 | 9 | 3.45 | 9 |
| 3.36 | 8 | | |
| 3.32 | 9 | 3.33 | 9 |
| 3.26 | 3 | 3.26 | 4 |

| | |
|---|---|
| 3.20 | 2 |
| 3.15 | 2 |
| 3.06 | 10 |
| 2.996 | 14 |
| 2.956 | 6 |
| 2.874 | 2 |
| 2.793 | 1 |
| 2.741 | 4 |
| 2.618 | 5 |
| 2.497 | 6 |
| 2.404 | 5 |
| 2.332 | 1 |
| 2.237 | 1 |
| 2.209 | 1 |
| 2.177 | 1 |
| 2.118 | 2 |
| 2.080 | 3 |
| 2.000 | 15 |
| 1.959 | 4 |
| 1.920 | 4 |
| 1.879 | 5 |
| 1.846 | 2 |
| 1.766 | 3 |
| 1.719 | 1 |
| 1.672 | 6 |
| 1.616 | 2 |
| 1.569 | 1 |

a = 20.10
b = 20.01
c = 13.49

| | |
|---|---|
| 3.20 | 2 |
| 3.15 | 2 |
| 3.07 | 11 |
| 2.997 | 14 |
| 2.955 | 6 |
| 2.876 | 2 |
| 2.799 | 1 |
| 2.742 | 4 |
| 2.682 | 1 |
| 2.615 | 5 |
| 2.570 | 2 |
| 2.498 | 7 |
| 2.403 | 5 |
| 2.333 | 1 |
| 2.239 | 1 |
| 2.209 | 1 |
| 2.179 | 1 |
| 2.079 | 2 |
| 2.001 | 13 |
| 1.957 | 3 |
| 1.923 | 4 |
| 1.881 | 5 |
| 1.774 | 2 |
| 1.719 | 1 |
| 1.667 | 6 |
| 1.626 | 2 |
| 1.615 | 2 |
| 1.569 | 1 |

a = 20.13
b = 20.03
c = 13.50

Example 3

The following solutions were prepared:

Solution A

4.9 g $Fe(NO_3)_3.9H_2O$

6.6 g $H_2SO_4$, conc

30.7 g $H_2O$

Solution B

7.0 g tetrapropylammonium bromide

16.4 g $H_2O$

9.7 g $NaSO_4.10H_2O$

Solution C:

The following mixture:

21.9 g acid-extracted silica

6.6 NaOH

96.1 g $H_2O$

was heated at 80°C until the silica was dissolved.

Solutions A and B were combined, then solution C was added with stirring. Finally 3.0 g of $Na_2SO_4.10H_2O$ and 5 g of 5N NaOH were added. The mixture was homogenized and then heated at 165°C. until the crystallization was complete (6 days).

The following analysis was carried out on the hydrogen form of the resultant ferrosilicate ("as crystallized" form calcined, $NH_4$+ exchanged and calcined).

86.4% $SiO_2$

170 ppm $Al_2O_3$

3.52% Fe

330 ppm Na

95.4% Ash

Comparative Example A

This example was an attempt to reproduce silicate 1 of Kouwenhoven U.S. Patent No. 4,238,318.

Sodium nitrate, 2.1 g, and 10.0 g of $Fe(NO_3)_3.9H_2O$ were dissolved in 9.6 g of water. To this solution was added a mixture of 72 g of colloidal silica sol (30% $SiO_2$) and 61.6 g of tetrapropylammonium hydroxide (35%). The latter was prepared by evaporating water from a commercial 25% solution. The composite reaction mixture was mixed thoroughly and heated to 150°C in a teflon-lined autoclave. After the designated time of 48 hours the mixture was still amorphous. Even after 64 hours no crystallization had occurred. The temperature was then raised to 160°C. After 62 hours at this temperature the mixture consisted mainly of 1-4 mm crystalline particles but still contained a considerable portion of gel. Finally, after a total of 184 hours (64 hours at 150°C and 120 hours at 160°C), the crystallization was terminated as no further improvement was observed. The product was washed to pH 8 and dried at 120°C.

The tan-colored material, which turned deep brown upon calcination, gave the x-ray diffraction pattern of ZSM-5 of 70% crystallinity.

Comparative Example B

This example was a repeat of silicate 2 of U.S. Patent 4,238, 318.

The reaction mixture had the same composition as that of Comparative Example A except that only half of the amount of ferric nitrate was used. The mixture crystallized in the designated time of 48 hours at 150°C.

The light-tan-colored product, which turned a purplish-brown upon calcination, gave the x-ray diffraction pattern of ZSM-5 of 90% crystallinity.

The ion-exchange capacities of the materials of Comparative Examples A and B were more than 30 times that attributable to the aluminum content. Aside from the observed colors, the (Na+N)/(Al+Fe) atomic ratios of the ammonium-exchanged forms indicate the presence of external iron oxide. This ratio was 0.48 for Comparative Example A and 0.77 for Comparative Example B.

The catalytic activity of external iron oxide disguises the activity associated with framework-iron. It is, therefore, desirable to produce a ZSM-5 type material containing no or only minor amounts of external iron oxide, but substantial amounts of iron substituting for framework-aluminum and/or framework silicon. In accordance with the invention, a new ZSM-5 type zeolite containing iron atoms in place of aluminum atoms in the crystal framework has been synthesized. It displays lower acid strength than the corresponding aluminosilicate ZSM-5 and after calcination, it exhibits no discoloration due to external iron oxide.

A series of experiments were conducted to compare the properties of Catalyst A, as prepared by the procedure of example 3 of the present invention, with Catalyst B, as Silicate 1 of the Kouwenhoven patent. These experiments were conducted using standard laboratory procedures, as set forth below.

a. Temperature programmed $NH_3$ desorption (TPAD) was carried out on both Catalyst A and Catalyst B using a DuPont 951 Thermogravimetric Analyzer equipped with an automatic titrimeter assembly, following the procedure of Kerr and Chester, Thermochim. Acta 3, 113 (1971). The exchange capacity of these zeolites are tabulated below.

| | CATALYST A | CATALYST B |
|---|---|---|
| Total Fe, wt.% | 3.7 | 6.5 |
| $T_{max}$, °C | 350 | 347 |
| Exchange capacity (meq/g ash) | 0.42 | 0.17 |
| $SiO_2/Fe_2O_3$ (framework) | 78 | 196 |

From the above data, it can be concluded that catalyst B has about 40% of the ammonia exchange capacity of Catalyst A, indicating a lesser amount of tetrahedrally coordinated (framework) Fe. This is calculated in the data above in the $SiO_2/Fe_2O_3$ molar ratio for framework Fe.

b. Electron Spin Resonance (ESR) spectra were recorded at 118°K on two samples of the above identified zeolites. Each spectrum exhibited a broad (delta H greater than 400 G) signal at g = 2.04 and a narrow signal at g = 4.3. These peaks are ascribed to non-framework and framework $Fe^{+3}$, respectively on the basis of literature. Itoh et al, J. Mol. Catal., 21, 151 (1983); McNicol et al J.

Catal., 25 223 (972). The intensity of the g = 2.04 signal was greater for the Catalyst A sample than for the Catalyst B sample whereas the intensity of the g=4.3 signal was greater for the Catalyst B sample than for the Catalyst A sample. These differences in intensity indicate that the Catalyst A sample exhibited a greater proportion of framework $Fe^{3+}$ relative to non-framework $Fe^{3+}$, than did the CATALYST B sample. Because of the absorptions resulting in non-level baseline these spectra could not be integrated to quantify the $Fe^{+3}$ in the zeolite framework.

Comparative Example C

An aluminosilicate was prepared according to U.S. Patent Re. 29,948 to provide an acidic ZSM-5 with 500 ppm $Al_2O_3$.

Comparative Example D

In a further reproduction of the teaching of U.S. Patent No. 4,238,318, 30.0g of dried silica gel was dissolved in 125.6g of 25% tetrapropylammonium hydroxide. A solution containing 13.87 g $Fe(NO_3)_3.9H_2O$ and 2.92 $NaNO_3$ in 47 g $H_2O$ was added. The mixture was homogenized and heated at 150°C. for 48 hours. The product obtained was crystalline and had a brown color.

A series of standard experimental olefinic conversion runs was conducted using a standard fixed bed of pelletized zeolite catalyst under substantially isothermal conditions at 230°C (446°F) and 10400 kPa (1500 psig), using each of the above identified catalysts. The results are set forth in Table 2 below.

TABLE 2

| RUN NO. | | 1 | 2 | 3 | 4 | 5 |
|---|---|---|---|---|---|---|
| Catalyst | = | FeZSM-5 of Example 3 Example D | FeZSM-5 of Comparative | FeZSM-5 of Example 3 | FeZSM-5 of Example 3 | (Al)ZSM-5 of Comparative Example C |
| Fe | = | 3.7 wt.% | 6.5 | 3.7 | 3.7 | nil |
| $Al_2O_3$ | = | 180 ppm | 860 | 180 | 180 | 500 |
| Feed | = | propene | propene | butene-1 | butene-1 | butene-1 |
| Space Vel. (LHSV) | = | 0.38 | 0.38 | 0.33 | 0.33 | 0.33 |
| Time on Stream (Hrs.) | = | 22 | 22 | 19 | 44 | 50 |
| Products (wt.%) | | | | | | |
| $C_1$-$C_5$ | | 1.1 | 76.8 | 3.4 | 2.2 | 93.4 |
| $C_6$-165°C | | | 22.7 | 14.4 | 37.4 | 38.2 |
| 2.4 | | | | | | |
| 165-345°C. | | | 64.9 | 7.7 | 51.2 | 51.8 |
| 2.2 | | | | | | |
| 345°F+ | | 11.3 | 1.0 | 7.7 | 7.8 | 2.0 |

One skilled in the art of olefin oligomerization by acidic zeolites containing Bronsted sites would attribute the improvement in oligomerization to increased concentration of framework Fe in the catalyst of the invention.

**Claims**

1. A crystalline iron containing silicate having the X-ray diffraction pattern of ZSM-5 and a crystallographic lattice containing silicon and iron, wherein at least 60% by weight of the iron contained by said silicate is in positions of tetrahedral substitution in the lattice and wherein the molar ratio of $SiO_2$ to $Fe_2O_3$ in said crystalline iron containing silicate is at least 30.

2. The crystalline iron-containing silicate of claim 1 wherein at least 80% by weight of the iron contained by the silicate is in positions of tetrahedral substitution.

3. The crystalline iron-containing silicate of claim 1, wherein said molar ratio is 30 to 500.

4. The crystalline iron-containing silicate of claim 1, wherein said molar ratio is 40 to 300.

5. A process for preparing the crystalline iron-containing silicate of claim 1 comprising the steps of:

(a) providing a crystallization reaction mixture which includes a source of $Fe_2O_3$, a source of $SiO_2$ and water, wherein said source of $Fe_2O_3$ comprises a complex of $Fe^{3+}$; and

(b) maintaining said crystallization reaction mixture at a temperature of 80°C to 200°C at autogenous pressure and maintaining said complex of $Fe^{+3}$in the complexed state until crystals of said ferrosilicate form.

6. The process of claim 5, wherein said crystallization reaction mixture further includes a source of an organic directing agent, $R_4J$, where R is an alkyl group containing 1-7 carbon atoms and J is an element of Group V-B.

7. The process of claim 6 wherein the organic directing agent is the tetrapropylammonium cation.

8. The process of claim 4, wherein the pH of the crystallization reaction mixture is maintained at from 9 to 13.

9. A hydrocarbon conversion process using as a catalyst the crystalline iron-containing silicate of any one of claims 1 to 4.

10. The process claimed in claim 9, wherein the conversion is oligomerization of an olefinic feedstock to produce heavier hydrocarbons.

11. The process of claim 10, wherein the feedstock contains $C_2$-$C_8$ olefins and is converted at a temperature of 175 to 375°C, a pressure of 1000 to 20000 kPa and 0.1 to 10 LHSV.